# EUROPEAN PATENT APPLICATION

(11) **EP 4 502 258 A1**
(43) Date of publication of application: **05.02.2025**
(21) Application number: 23781241.7
(22) Date of filing: 23.03.2023
(51) Int. Cl.: D04H 1/4266, D04H 1/728, D04H 1/485, A45D 44/00, A61K 8/02, A61K 8/73, A61Q 19/00, A61K 9/70, A61K 47/36

(54) **HYALURONATE NANOFABRIC SHEET AND MANUFACTURING METHOD THEREFOR**

(30) Priority: 31.03.2022 KR 20220040033; 14.06.2022 KR 20220072058
(71) Applicant: Jinwoo Bio Co., Ltd., Giheung-gu Yongin-si, Gyeonggi-do 17111 (KR)
(72) Inventor: KWEON, Dong Keon, Yongin-si Gyeonggi-do 16918 (KR); LEE, Myoung Han, Seoul 02799 (KR); KIM, Jong Soo, Bucheon-si Gyeonggi-do 14539 (KR); HONG, Joo Yeon, Guri-si, Gyeonggi-do 11920 (KR)
(74) Representative: Ter Meer Steinmeister & Partner
(86) International application number: PCT/KR2023/003879
(87) International publication number: WO 2023/191385

(57) **Abstract**

The present invention relates to a hyaluronate nanofabric sheet and a manufacturing method therefor and, more particularly, to a hyaluronate nanofabric sheet having excellent percutaneous absorption and physical properties and a manufacturing method therefor. A method for manufacturing a hyaluronate nanofabric sheet according to the present invention comprises the steps of: (a) mixing a hyaluronate having a weight average molecular weight of 5×10³ - 5×10⁴ Da and a hyaluronate having a weight average molecular weight of 1×10⁵ - 1×10⁶Da and dissolving same in water to prepare an aqueous hyaluronate solution; and (b) electrospinning the aqueous hyaluronate solution. The hyaluronate nanofabric sheet of the present invention has an excellent transdermal absorption rate due to the ultra-low molecular weight hyaluronic acid as a main component thereof and exhibits excellent physical properties due to a certain amount of medium-molecular weight hyaluronic acid mixed therewith, and as such, can be applied for various purposes, such as mask patches for topical uses, ophthalmic or mucosal drug delivery systems, etc.

## Description

### Technical Field

The present disclosure relates to a hyaluronate nanofabric sheet and a method of manufacturing the same and, more particularly, to a hyaluronate nanofabric sheet having an excellent transdermal absorption rate and physical properties, and a method of manufacturing the same.

### Background Art

Hyaluronic acid or hyaluronate, a biocompatible material that is evenly distributed throughout connective, epithelial, and nervous tissues in the human body and has a variety of physiological activities, can contain a large amount of water and has excellent viscoelasticity, thus being proven to be effective in regenerating and moisturizing the skin, maintaining skin elasticity, and reducing wrinkles. Accordingly, there has recently been a rapidly growing demand for anti-aging cosmetics, food, medicine, medical devices, and dermal fillers containing hyaluronic acid or hyaluronate.

Finished products using hyaluronate currently available in the market, mostly in liquid form, contain hyaluronate at low concentrations, that is, such products are primarily composed of water rather than hyaluronate, making the use thereof limited. Moreover, these products must be manufactured in aseptic facilities for use in medicine or medical devices due to the low safety of hyaluronate itself against microorganisms, and must involve the use of preservatives for safety concerns or use in cosmetics.

Therefore, research has been conducted on transforming hyaluronic acid or hyaluronate into solids, such as films and fibers, other than liquids.

Korean Patent Application Publication No. 2019-0138907 disclosed a mask sheet including a hyaluronic acid film, and Korean Patent Application Publication No. 2019-0106091 disclosed a method of manufacturing a hyaluronate film by a solvent casting method or a casting method using an automatic film applicator.

However, the manufacturing process of hyaluronate films is rather complicated when mass-produced, and there are problems with a low transdermal absorption rate and poor adhesion to the skin when used as facial masks and the like.

To improve such a manufacturing process of hyaluronate films, Korean Patent No. 2338355 disclosed a method of manufacturing a wet hyaluronate non-woven fabric usable as facial patches, wet dressings, tissue anti-adhesion barriers, and the like, and Korean Patent No. 1224882 disclosed a nanofabric sheet composed of hyaluronic acid. However, Korean Patent No. 2338355 involved the use of hyaluronate having a molecular weight in the range of 50,000 to 100,000 daltons (Da), and Korean Patent No. 1224882 involved the use of hyaluronate of 100,000 to 1,600,000 Da. Thus, there has been a problem with a low transdermal absorption rate.

Hyaluronic acid or hyaluronate can be categorized into ultra-low-molecular-weight, low-molecular-weight, medium-molecular-weight, and high-molecular-weight forms, depending on the molecular weight thereof.

Medium-molecular-weight and high-molecular-weight hyaluronates are easily manufactured into film forms due to having excellent mechanical properties but are poorly penetrated and absorbed into the skin. On the other hand, the lower the molecular weight of hyaluronic acid or hyaluronate, the easier the penetration and absorption into the skin. However, due to poor mechanical properties, lower-molecular-weight hyaluronic acid or hyaluronate is poorly manufactured into a film form, which is problematic.

In addition, hyaluronate is a high-molecular-weight polysaccharide containing many hydroxyl groups in the molecule thereof, and the molecule itself is positively charged, so there are many limitations in electrospinning. Thus, in the case of manufacturing hyaluronate non-woven fabrics by existing electrospinning (Castro, K. C., Campos, M. G. N., & Mei, L. H. I. (2021). International Journal of Biological Macromolecules, 173, 251-266.), hyaluronic acid non-woven fabrics were manufactured using alkaline solvents such as sodium hydroxide or potassium hydroxide or toxic organic solvents (Ewelina, et al. Advances in Polymer Technology 37.6 (2018): 1929-1940.). However, toxic organic solvents or salts must be used, and there is a concern that such organic solvents or salts remain after forming non-woven fabrics, thus limiting the practical use thereof.

Hence, the inventors of the present disclosure have made efforts to manufacture a hyaluronic acid sheet that is easily penetrated and absorbed into the skin and has excellent mechanical properties for commercialization, without the addition of organic solvents, salts, and the like. As a result, the inventors of the present disclosure have confirmed that a hyaluronate nanofabric sheet having an excellent transdermal absorption rate and mechanical properties can be manufactured by using an ultra-low-molecular-weight hyaluronic acid of 5,000 to 5 × 10⁴ Da serving as a main component and being easily penetrated and absorbed into the skin, adding a medium-molecular-weight hyaluronate of 1 × 10⁵ to 1 × 10⁶ Da having excellent mechanical properties that can provide processability for commercialization, dissolving the resulting product in water, and electrospinning the dissolved product, thereby completing the present disclosure.

### Disclosure

### Technical Problem

The present disclosure aims to provide a hyaluronate nanofabric sheet having an excellent transdermal absorption rate and mechanical properties.

The present disclosure also aims to provide a method of manufacturing a hyaluronate nanofabric sheet having an excellent transdermal absorption rate and tensile strength.

### Technical Solution

To achieve the objectives described above, the present disclosure provides a hyaluronate nanofabric sheet manufactured by electrospinning using purified water as a single solvent, the hyaluronate nanofabric sheet containing hyaluronate fibers having a diameter in the range of 0.3 to 5 µm.

To achieve the objectives described above, the present disclosure provides a hyaluronate nanofabric sheet having a transdermal absorption rate in the range of 77.70% to 86.08% and a tensile strength in the range of 5.32 to 10.91 MPa.

In the present disclosure, the hyaluronate of the hyaluronate nanofabric sheet is characterized by including 88.9 to 98.1 wt% of a hyaluronate having a weight average molecular weight in the range of 5 × 10³ to 5 × 10⁴ Da and 1.9 to 11.1 wt% of a hyaluronate having a weight average molecular weight in a range of 1 × 10⁵ to 1 × 10⁶ Da.

In the present disclosure, the hyaluronate sheet is characterized by being used as a mask patch for external skin application and an ophthalmic or mucosal drug delivery system carrier.

The present disclosure also provides a method of manufacturing a hyaluronate nanofabric sheet, the method being characterized by including the following steps: (a) mixing a hyaluronate having a weight average molecular weight in the range of 5 × 10³ to 5 × 10⁴ Da and a hyaluronate having a weight average molecular weight in the range of 1 × 10⁵ to 1 × 10⁶ Da and dissolving the resulting mixture in water to prepare an aqueous hyaluronate solution; and (b) electrospinning the aqueous hyaluronate solution.

In the present disclosure, the aqueous hyaluronate solution is characterized by having a viscosity in the range of 1,000 to 15,000 cPs.

### Advantageous Effects

A hyaluronate nanofabric sheet of the present disclosure not only is primarily composed of an ultra-low-molecular-weight hyaluronic acid, thus having an excellent transdermal absorption rate but also exhibits excellent mechanical properties due to a certain amount of a medium-molecular-weight hyaluronic acid mixed therein.

### Description of Drawings

FIG. 1 shows images of a hyaluronate nanofabric sheet manufactured by electrospinning according to one embodiment of the present disclosure (A: nanofiber-structure sheet, B: dot-structure sheet, C: micrograph of nanofiber-structure sheet, D: micrograph of dot-structure sheet); and
FIG. 2 shows images of a transmission experiment performed on a hyaluronate nanofabric sheet manufactured according to one embodiment of the present disclosure, using a fluorescence microscope.

### Best Mode

As used herein, the term "hyaluronate nanofabric sheet" refers to a hyaluronate nanofiber-containing sheet manufactured by electrospinning.

The lower the molecular weight of hyaluronic acid or hyaluronate, the more easily hyaluronic acid or hyaluronate is penetrated and absorbed into the skin, but the more challenging it is to manufacture sheets with good physical properties by electrospinning, which is problematic.

Korean Patent No. 2338355 involved the use of hyaluronate having a molecular weight in the range of 50,000 to 100,000 Da to manufacture a hyaluronate non-woven fabric, and Korean Patent No. 1224882 involved the use of hyaluronate of 100,000 to 1,600,000 Da to manufacture a nanofabric sheet composed of hyaluronic acid. In the cases above, high-molecular-weight hyaluronate is used, so alkaline solvents such as sodium hydroxide and potassium hydroxide are used to reduce viscosity. In addition, the transdermal absorption rate is low, so a variety of physiological activities of hyaluronic acid are poorly delivered to the skin, which is problematic.

The present disclosure aimed to confirm that a hyaluronate nanofabric sheet having not only an excellent transdermal absorption rate but also excellent mechanical properties enough to be processible was able to be uniformly mass-produced when adding a medium-molecular-weight hyaluronate of 1 × 10⁵ to 1 × 10⁶ Da to an ultra-low-molecular-weight hyaluronic acid 5 × 10³ to 5 × 10⁴ Da.

In the present disclosure, the hyaluronate nanofabric sheet was manufactured by mixing an ultra-low-molecular-weight hyaluronate and a medium-molecular-weight hyaluronate, dissolving the resulting mixture in purified water alone, without the addition of an alkaline solvent to prepare an aqueous hyaluronate solution having a viscosity in the range of 1,000 to 15,000 cPs (25°C), and electrospinning the aqueous hyaluronate solution.

As a result, the hyaluronate nanofabric sheet was able to be manufactured in large quantities, and the transdermal absorption rate of the hyaluronate nanofabric sheet manufactured was confirmed to be in the range of 77.70% to 86.08%.

Accordingly, in one aspect, the present disclosure relates to a hyaluronate nanofabric sheet manufactured by electrospinning using purified water as a single solvent, the hyaluronate nanofabric sheet containing hyaluronate fibers having a diameter in the range of 0.3 to 5 µm.

In addition, in another aspect, the present disclosure relates to a hyaluronate nanofabric sheet having a transdermal absorption rate in the range of 77.70% to 86.08% and a tensile strength in the range of 5.32 to 10.91 MPa.

The hyaluronate constituting the hyaluronate nanofabric sheet is characterized by including 88.9 to 98.1 wt% of a hyaluronate having a weight average molecular weight in the range of 5 × 10³ to 5 × 10⁴ Da and 1.9 to 11.1 wt% of a hyaluronate having a weight average molecular weight in a range of 1 × 10⁵ to 1 × 10⁶ Da.

In other words, the hyaluronate nanofabric sheet of the present disclosure is characterized in that because sheets with good physical properties are unlikely to be formed by electrospinning using the ultra-low-molecular-weight hyaluronate alone, the medium-molecular-weight hyaluronate is used in conjunction to form the skeleton of the sheet.

In the present disclosure, the molecular weight of the ultra-low-molecular-weight hyaluronate is in the range of 5 × 10³ to 5 × 10⁴ Da, and the molecular weight of the medium-molecular-weight hyaluronate is in the range of 1 × 10⁵ to 1 × 10⁶ Da.

When the content of the hyaluronic acid having a molecular weight in the range of 5 × 10³ to 5 × 10⁴ Da is less than 88.9 wt%, the sheet fails to be manufactured, or the transdermal absorption rate is reduced. When the content of the aforementioned hyaluronic acid exceeds 98.1 wt%, the sheet is formed, but the physical properties, such as tensile strength, deteriorate, which is problematic.

In the present disclosure, examples of the hyaluronates having a form in which a salt is bound to hyaluronic acid may include sodium hyaluronate, calcium hyaluronate, potassium hyaluronate, and the like but are not limited thereto.

In a further aspect, the present disclosure provides a method of manufacturing a hyaluronate nanofabric sheet, the method being characterized by including the following steps: (a) mixing a hyaluronate having a weight average molecular weight in the range of 5 × 10³ to 5 × 10⁴ Da and a hyaluronate having a weight average molecular weight in the range of 1 × 10⁵ to 1 × 10⁶ Da and dissolving the resulting mixture in water to prepare an aqueous hyaluronate solution; and (b) electrospinning the aqueous hyaluronate solution.

In the present disclosure, the weight ratio of the hyaluronic acid having a weight average molecular weight in the range of 5 × 10³ to 5 × 10⁴ Da to the hyaluronic acid having a weight average molecular weight in the range of 1 × 10⁵ to 1 × 10⁶ Da is preferably in the range of 88.9 to 98.1 wt%:1.9 to 11.1 wt%.

In the present disclosure, the hyaluronate solution in which the ultra-low-molecular-weight and medium-molecular-weight hyaluronic acids are mixed preferably has a viscosity in the range of 1,000 to 15,000 cPs (25°C) (Brookfield DV2RTVJO, Spindle No. 5, 12 rpm). While the viscosity of the solution may vary with the molecular weight (Da) of hyaluronic acid (HA), HA content, and the ratio with respect to the solvent, when the viscosity is less than 1,000 cPs, sheet formation is challenging, and when the viscosity exceeds 15,000 cPs, there is a concern that electrospinning may be poorly performed because the nozzle is clogged due to the high viscosity.

In the present disclosure, the electrospinning is preferably performed under the following conditions: a tension in the range of 30 to 35 kV, a flow rate in the range of 10 to 20 mL/h, and a distance in the range of 10 to 15 cm.

In the present disclosure, the hyaluronate nanofabric sheet manufactured by the electrospinning may contain hyaluronate fibers having a diameter in the range of 0.3 um to 5 µm.

In the present disclosure, purified water is preferably used as the solvent to dissolve the hyaluronate.

In addition, the electrospun hyaluronate nanofabric sheet used in the present disclosure may be manufactured using hyaluronate alone, but may additionally include other watersoluble, low-molecular-weight carrier or excipient components commonly used in the art depending on the field of application, and these types and content ranges are not particularly limited.

### Mode for Invention

Hereinafter, the present disclosure will be described in more detail through examples. These examples are only for illustrating the present disclosure, and it will be apparent to those skilled in the art that the scope of the present disclosure is not to be construed as being limited by these examples.

### Example 1: Manufacturing of hyaluronate nanofabric sheet by electrospinning

Using an electrospinning machine (NE300, Inovenso Co., Turkey), the tension, flow rate, and distance were set as shown in Table 1 below, and then spinning was performed by varying the hyaluronate molecular weight and aqueous solution concentration conditions.

**[Table 1]**

| Example | Molecular Weight | Aqueous Solution Concentration (Wt.%) | Viscosity* (cPs) | Electrospinning Condition | | | Sheet being formed or not |
|---|---|---|---|---|---|---|---|
| | | | | Tension (kV) | Flow Rate (mL/h) | Distance (cm) | |
| 1-1 | 5 kDa | 10 | 430 | 30 | 10 to 30 | 10 to 15 | X |
| | | | | 35 | | | X |
| 1-2 | | 20 | 1080 | 30 | 10 to 30 | 10 to 15 | ○ |
| | | | | 35 | | | ○ |
| 1-3 | | 30 | 2610 | 30 | 10 to 30 | 10 to 15 | ○ |
| | | | | 35 | | | ○ |
| 1-4 | 10 kDa | 10 | 760 | 30 | 10 to 30 | 10 to 15 | X |
| | | | | 35 | | | X |
| 1-5 | | 20 | 1750 | 30 | 10 to 30 | 10 to 15 | ○ |
| | | | | 35 | | | ○ |
| 1-6 | | 30 | 4280 | 30 | 10 to 30 | 10 to 15 | ○ |
| | | | | 35 | | | ○ |
| 1-7 | 50 kDa | 5 | 11,910 | 30 | 10 to 30 | 10 to 15 | ○ |
| | | | | 35 | | | ○ |
| 1-8 | | 10 | 16,610 | 30 | 10 to 30 | 10 to 15 | X |
| | | | | 35 | | | X |
| 1-9 | | 20 | 22,820 | 30 | 10 to 30 | 10 to 15 | X |
| | | | | 35 | | | X |
| 1-10 | 0.1 MDa | 1 | 80 | 30 | 10 to 30 | 10 to 15 | X |
| | | | | 35 | | | X |
| 1-11 | | 5 | 48,740 | 30 | 10 to 30 | 10 to 15 | X |
| | | | | 35 | | | X |
| 1-12 | | 10 | 87,210 | 30 | 10 to 30 | 10 to 15 | X |
| | | | | 35 | | | X |

| | | | | | | | |
|---|---|---|---|---|---|---|---|
| *Viscosity values were measured using a viscometer (DV2TRV TJO, Brookfield Engineering Labs Inc., USA) under the following conditions: 12 rpm, 30 seconds, and 25°C. From Table 1, it was confirmed that sheet formation by electrospinning was possible under the molecular weight condition in the range of 5 kDa to 50 kDa. In addition, it was seen that sheets with poor physical properties were manufactured because the nanofiber-structure sheets with good physical properties partially contained a dot structure depending on the concentration or molecular weight of the hyaluronates (FIG. 1). | | | | | | | |

### Example 2: Manufacturing of hyaluronate nanofabric sheet by electrospinning

Electrospinning was performed in the same manner as in Example 1 using aqueous hyaluronate solution compositions shown in Table 2 under the following conditions: a tension of 30 kV, a flow rate in the range of 10 to 30 mL/h, and a distance in the range of 10 to 15 cm.

**[Table 2]**

| Example | Ultra-low-molecular-weight | | Medium-molecular-weight | | HA solution viscosity (cPs) | Sheet being formed or not |
|---|---|---|---|---|---|---|
| | HA molecular weight (Da) | Wt% | HA molecular weight (Da) | Wt% | | |
| 2-1 | 5,000 | 100 | - | - | 1,080 | ○ |
| 2-2 | 5,000 | 88.9 | 1 × 10⁵ | 11.1 | 7,400 | ○ |
| 2-3 | 5,000 | 81.5 | 1 × 10⁵ | 18.5 | 16,100 | X |
| 2-4 | 5,000 | 88.9 | 1 × 10⁶ | 11.1 | 12,400 | ○ |
| 2-5 | 5,000 | 81.5 | 1 × 10⁶ | 18.5 | 24,700 | X |
| 2-6 | 10,000 | 100 | - | - | 1,750 | ○ |
| 2-7 | 10,000 | 98.1 | 1 × 10⁵ | 1.9 | 5,300 | ○ |
| 2-8 | 10,000 | 96.3 | 1 × 10⁵ | 3.7 | 6,233 | ○ |
| 2-9 | 10,000 | 98.1 | 1 × 10⁶ | 1.9 | 8,400 | ○ |
| 2-10 | 10,000 | 96.3 | 1 × 10⁶ | 3.7 | 13,240 | ○ |
| 2-11 | 50,000 | 100 | - | - | 11,910 | ○ |
| 2-12 | 50,000 | 98.1 | 1 × 10⁵ | 1.9 | 14,700 | ○ |
| 2-13 | 50,000 | 96.3 | 1 × 10⁵ | 3.7 | 21,300 | X |
| 2-14 | 50,000 | 98.1 | 1 × 10⁶ | 1.9 | 19,700 | X |

From Table 2, it was seen that when using the hyaluronate of 5 × 10³ Da, 1 × 10⁴ Da, or 5 × 10⁴ Da alone, or in the case where the content of the hyaluronate having a weight average molecular weight in the range of 5 × 10³ to 5 × 10⁴ Da was in the range of 88.9 to 98.1 wt%, the content of the hyaluronate having a weight average molecular weight in the range of 1 × 10⁵ to 1 × 10⁶ Da was in the range of 1.9 to 11.1 wt%, and the viscosity of the solution was in the range of 1,000 to 15,000 cPs, the hyaluronate nanofabric sheet was able to be formed.

### Experimental Example 1. Confirmation of microstructure of hyaluronate nanofabric sheet

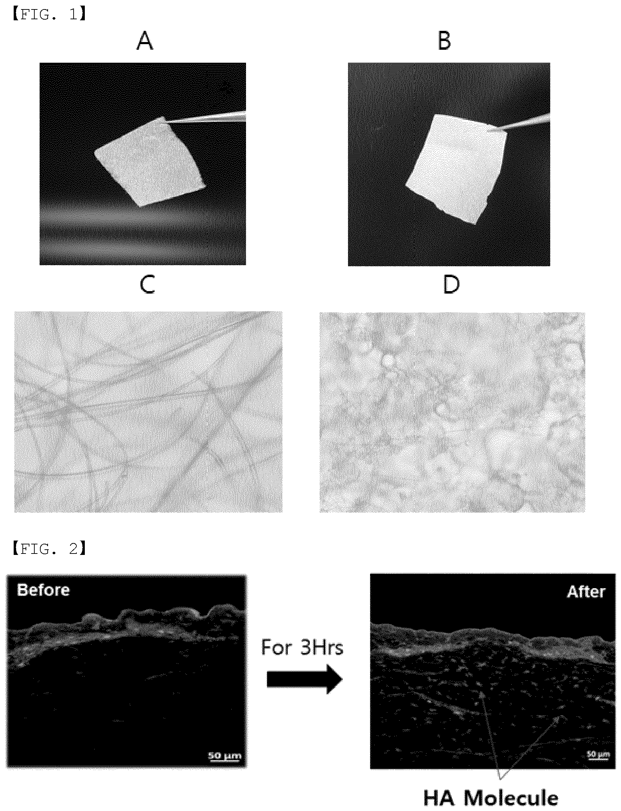

The microstructures of the electrospun hyaluronate nanofabric sheets were confirmed using a microscope (CX33RTFS2, Olympus Corporation., Korea) (FIG. 1). Depending on the molecular weight and concentration of hyaluronates, either a nanofiber structure with good physical properties (Example 2-4) or a dot structure with poor physical properties (Examples 2-6 and 2-11) was confirmed in the hyaluronate nanofabric sheets.

### Experimental Example 2. Transdermal absorption rate evaluation and tensile strength measurement

For a transdermal absorption rate test, a skin penetration test was conducted under sink conditions using a Franz diffusion cell (effective area: 0.64 cm², volume of receptor chamber: 5 mL) system (Logan Instruments, New Jersey, USA) according to the "Guidelines for in vivo skin absorption testing".

A aqueous phase used for the skin penetration test was phosphate-buffered saline (PBS) with a pH of 7.4. An epidermal layer (1.5 cm × 1.5 cm) of prepared human skin was positioned such that the stratum corneum was placed on the receptor chamber while facing upward and then covered with the donor chamber to hold the assembly with a clamp. Subsequently, the Franz diffusion cell was filled with the aqueous phase, and the temperature thereof was maintained within 32 ± 1°C to uniformly apply 100 µL of each hyaluronic acid sheet solution on the donor site, thereby conducting the penetration test. After conducting the penetration test, 5 ml of the aqueous phase was collected when 1, 3, 6, 9, 12, and 24 hours elapsed, analyzed, and replenished with fresh PBS.

Reference was made to the methods of Fudala, Rafal, et al. and de Belder, Anthony N. et al. (Photobiology B: Biology 104.3 (2011), Carbohydrate Research 44.2 (1975)) for additional qualitative analysis. It was confirmed by a fluorescence microscope that under the same penetration test conditions, the hyaluronic acid non-woven fabric (Example 2-4) was penetrated into the skin when three hours elapsed (FIG. 2) .

For tensile strength measurement, the tensile strength of the hyaluronate sheets manufactured in Example 2 was measured. The results thereof are shown in Table 3. Each sample with a length of 15 cm and a width of 2.5 cm was mounted on a test jig, and a crosshead speed was set as 2 mm/min to measure the tensile strength using a universal testing machine.

**[Table 3]**

| Example | HA molecular weight and content | Tensile strength (MPa) | Transdermal absorption rate |
|---|---|---|---|
| 2-1. | Ultra-low molecular weight (5,000 Da) 100 wt% | 2.14 | 92.81% |
| 2-2. | Ultra-low molecular weight (5,000 Da) 88.9 wt%, medium-molecular weight (1 × 10⁵ Da) 11.1 wt% | 8.43 | 86.08% |
| 2-4 | Ultra-low molecular weight (5,000 Da) 88.9 wt%, medium-molecular weight (1 × 10⁶ Da) 11.1 wt% | 10.91 | 83.11% |
| 2-6 | Ultra-low molecular weight (10,000 Da) 100 wt% | 2.52 | 90.72% |
| 2-7 | Ultra-low molecular weight (10,000 Da) 98.1 wt%, medium-molecular weight (1 × 10⁵ Da) 1.9 wt% | 5.32 | 84.65% |
| 2-8 | Ultra-low molecular weight (10,000 Da) 96.3 wt%, medium-molecular weight (1 × 10⁵ Da) 3.7 wt% | 8.36 | 80.97% |
| 2-9 | Ultra-low molecular weight (10,000 Da) 98.1 wt%, medium-molecular weight (1 × 10⁶ Da) 1.9 wt% | 6.07 | 81.66% |
| 2-10 | Ultra-low molecular weight (10,000 Da) 96.3 wt%, medium-molecular weight (1 × 10⁶ Da) 3.7 wt% | 9.13 | 78.23% |
| 2-11 | Ultra-low molecular weight (50,000 Da) 100 wt% | 3.08 | 87.34% |
| 2-12 | Ultra-low molecular weight (50,000 Da) 98.1 wt%, medium-molecular weight (1 × 105 Da) 1.9 wt% | 8.49 | 77.70% |

The hyaluronate nanofabric sheets manufactured using the hyaluronate of 5 × 10³ Da, 1 × 10⁴ Da, or 5 × 10⁴ Da alone have transdermal absorption rates in the range of 87.34% to 92.81%, meaning that the transdermal absorption rates are high. However, the tensile strength measurement values are in the range of 2.14 to 3.08 MPa, resulting in poor physical properties. Thus, there have been problems with the sheets being damaged during post-processing, such as blanking and packaging. On the other hand, in the case of the hyaluronate nanofabric sheets manufactured by mixing the ultra-low-molecular-weight and medium-molecular-weight hyaluronic acids, the transdermal absorption rates are in the range of 77.70% to 86.08%, and the tensile strength measurement values were in the range of 5.32 to 10.91 MPa, resulting in an excellent transdermal absorption rate and physical properties. Thus, such hyaluronate nanofabric sheets can be effectively used as mask patches for external skin applications and ophthalmic or mucosal drug delivery system carriers.

In particular, it was confirmed that the lower the molecular weight of the medium-molecular-weight hyaluronate mixed, the higher the transdermal absorption rate, but the lower the tensile strength.

Specific aspects of the present disclosure have been described in detail above, and those skilled in the art will appreciate that these specific aspects are only preferred embodiments, and the scope of the present disclosure is not limited thereby. Thus, the substantial scope of the present disclosure will be defined by the appended claims and their equivalents.

### Industrial Applicability

The hyaluronate nanofabric sheet of the present disclosure also has excellent mechanical properties and, therefore, can be applied for various purposes, such as mask patches for external skin applications and ophthalmic or mucosal drug delivery system carriers.

## Claims

1. A hyaluronate nanofabric sheet manufactured by electrospinning using purified water as a single solvent, the hyaluronate nanofabric sheet comprising hyaluronate fibers having a diameter in a range of 0.3 to 5 µm.

2. A hyaluronate nanofabric sheet having a transdermal absorption rate in a range of 77.70% to 86.08% and a tensile strength in a range of 5.32 to 10.91 MPa.

3. The hyaluronate nanofabric sheet of claim 2, wherein the hyaluronate of the hyaluronate nanofabric sheet comprises 88.9 to 98.1 wt% of a hyaluronate having a weight average molecular weight in a range of 5 × 10³ to 5 × 10⁴ Da and 1.9 to 11.1 wt% of a hyaluronate having a weight average molecular weight in a range of 1 × 10⁵ to 1 × 10⁶ Da.

4. The hyaluronate nanofabric sheet of claim 2, wherein the hyaluronate nanofabric sheet is used as a mask patch for external skin application and an ophthalmic or mucosal drug delivery system carrier.

5. A method of manufacturing a hyaluronate nanofabric sheet, the method comprising:
(a) mixing a hyaluronate having a weight average molecular weight in a range of 5 × 10³ to 5 × 10⁴ Da and a hyaluronate having a weight average molecular weight in a range of 1 × 10⁵ to 1 × 10⁶ Da and dissolving the resulting mixture in water to prepare an aqueous hyaluronate solution; and
(b) electrospinning the aqueous hyaluronate solution.

6. The method of claim 5, wherein a weight ratio of the hyaluronate having a weight average molecular weight in a range of 5 × 10³ to 5 × 10⁴ Da to the hyaluronate having a weight average molecular weight in a range of 1 × 10⁵ to 1 × 10⁶ Da is in a range of 88.9 to 98.1 wt%:1.9 to 11.1 wt%.

7. The method of claim 5, wherein the aqueous hyaluronate solution has a viscosity in a range of 1,000 to 15,000 cPs.
